# EUROPÄISCHE PATENTSCHRIFT

(11) **EP 4 570 288 B1**
(45) Veröffentlichungstag und Bekanntmachung des Hinweises auf die Patenterteilung: **08.07.2026**
(21) Anmeldenummer: 24217378.9
(22) Anmeldetag: 04.12.2024
(51) Int. Cl.: A61M 16/00, A61M 16/08

(54) **ANORDNUNG ZU EINER REDUZIERUNG VON GERÄUSCHEN**
ARRANGEMENT FOR REDUCING NOISE
DISPOSITIF POUR RÉDUIRE LE BRUIT

(30) Priorität: 12.12.2023 DE 102023134721
(43) Veröffentlichungstag der Anmeldung: 18.06.2025
(73) Patentinhaber: Drägerwerk AG & Co. KGaA, 23558 Lübeck (DE)
(72) Erfinder: Caspary, René-Christian, 23558 Lübeck (DE)

(56) Entgegenhaltungen:
- EP-A1- 4 454 689
- WO-A1-2005/097244
- WO-A1-2022/094655
- WO-A1-2023/283001
- DE-A1- 102020 001 389

## Beschreibung

Die vorliegende Erfindung betrifft eine Anordnung zu einer Reduzierung von Geräuschen an einem Gaseinlass eines Gebläsebeatmungsgerätes. Solcherlei Anordnungen können auch als Schalldämpfer oder Einlassschalldämpfer bezeichnet werden.

Schalldämpfer sind aus vielen Bereichen der Technik bekannt, etwa zur Verminderung von Ansauggeräuschen an einem Verbrennungsmotor, beispielsweise für Kraftfahrzeuge, Schiffe oder auch für stationäre Motoren in Blockheizkraftwerken.

Staubsauger weisen zuweilen auch Komponenten zur Schalldämpfung auf. Eine Schalldämpfung im oder am Einlassbereich von Gebläsen ist wesentlich, um Schallabstrahlungen auch im niedrigen Lastbereich oder Teillastbereich zu reduzieren.

### Stand der Technik

Aus dem Stand der Technik sind Möglichkeiten zur Verminderung von Geräusch- oder Schallabstrahlungen bei Beatmungsgeräten bekannt.

So zeigt beispielsweise die US2008257347 AA eine Schalldämmung durch die Auskleidung mit Akustikschaum.

Auch Ausgestaltungen aus Akustikschaum im Einlassbereich sind realisierbar, wie beispielsweise durch die EP 2739857 B1 gezeigt. WO2023/283001 A1 offenbart einen Inline-Muffler für ein Positiv-Atemwegs-Druck-System, der zwischen dem Gebläse und dem Schlauch angebracht wird und über ein vergrößertes Expansions-Kammer-Gehäuse mit integrierten Baffles (z. B. Chevron- und Planar-Baffles) verfügt, um Schallenergie des Luftstroms zu absorbieren und den Geräuschpegel am Benutzerinterface zu reduzieren.

Für einen längerfristigen Betrieb von Gebläsebeatmungsgeräten im Krankenhaus, beispielsweise über mehr als 5 bis 10 Jahre hinaus, können Elemente mit Akustikschaum hinsichtlich der Hygiene und/oder der Materialalterung das Erfordernis mit sich bringen, diese Elemente im Rahmen regelmäßiger Wartungen zu überprüfen und ggf. auszutauschen, wenn eine maximale Einsatzdauer erreicht ist oder auch die mechanische Stabilität der Schaumstruktur nachgelassen hat.

Aus dem Blickwinkel von Personen, welche als Wartungspersonal die Betriebsbereitschaft des Gebläsebeatmungsgerätes sicherstellen müssen, ist solch ein Austausch von Komponenten mit Aufwand an Zeitplanung, Logistik im technischen Bereich der Instandhaltung und auch im kaufmännischen Bereich, etwa im Einkauf, verbunden.

Daraus ergibt sich die Motivation, ein Gebläsebeatmungsgerät zu entwickeln, welches möglichst frei von einer Vielzahl an Wartungs- oder Austauschteilen und insbesondere ohne Elemente mit Akustikschaum konstruierbar ist.

Im Lichte des Standes der Technik hat sich die vorliegende Erfindung die Aufgabe gestellt, eine Möglichkeit einer Minderung von Geräuschen an einem Gebläsebeatmungsgerät bereitzustellen.

Die Aufgabe zu einer Angabe einer Geräuschminderung für ein Gebläsebeatmungsgerät wird gelöst durch eine Anordnung zur Reduzierung von Geräuschen mit den Merkmalen des Patentanspruchs 1.

Vorteilhafte Ausführungsformen der Erfindung ergeben sich aus den Unteransprüchen und werden in der folgenden Beschreibung unter teilweiser Bezugnahme auf die Figuren näher erläutert.

Der Grundgedanke der Erfindung besteht darin, im Unterschied zu Lösungen, bei denen weiche Strukturen wie Schaumstoffe oder vergleichbare Materialien zu Verminderung von Betriebsgeräuschen oder Schall zum Einsatz kommen, durch eine Ausgestaltung der Führung von Luftmengen in festen Strukturen eine Verminderung einer Abstrahlung von Betriebsgeräuschen an die Umgebung zu erzielen.

Die erfindungsgemäße Anordnung zur Reduzierung von Geräuschen kann an einem Gaseinlass eines Gebläsebeatmungsgerätes angeordnet werden. Die Anordnung weist erfindungsgemäß ein Verbindungselement und mindestens ein Labyrinthelement auf. Das Labyrinthelement weist eine Labyrinthstruktur auf, welche einen Rauminhalt des Labyrinthelementes zu mindestens 40% ausfüllt. Die Labyrinthstruktur bildet eine Vielzahl von parallelen Kanälen mit Umlenkungen in dem mindestens einen Labyrinthelement aus. Die parallelen Kanäle sind zu einer Führung von Luftmengen und zu einer Beeinflussung einer Schallausbreitung ausgebildet. Das Verbindungselement weist einen Gasauslass auf, welcher mit einem Gaseingang des Gebläsebeatmungsgerätes verbindbar ist. Das Verbindungselement weist einen Gaseinlass zu einer Einströmung von Luftmengen aus einer Umgebung auf. Das mindestens eine Labyrinthelement weist mindestens eine Einlasskammer zu einer Aufnahme von Einlassluftmengen vom Verbindungselement auf. Das mindestens eine Labyrinthelement weist mindestens eine Auslasskammer zu einer Bereitstellung von Einlassluftmengen an das Verbindungselement auf.

Das Verbindungselement ist mit dem mindestens einen Labyrinthelement derart verbunden, dass die Luftmengen aus der Umgebung in die Labyrinthstruktur als Einlassluftmengen einströmen, die Vielzahl an Kanälen des mindestens einen Labyrinthelementes durchströmen und als Auslassluftmengen über das Verbindungselement und den Gasauslass des Verbindungselementes als Atemluftmengen in den Gaseingang des Gebläsebeatmungsgerätes einströmen.

Die Beeinflussung der Schallausbreitung wird durch die Labyrinthstruktur im Labyrinthelement in Verbindung mit dem Verbindungselement erzielt. Die Beeinflussung bewirkt, dass die Abstrahlung von Betriebsgeräuschen an die Umgebung, die durch den Gebläseantrieb des Gebläsebeatmungsantriebs sowohl bei großen wie auch bei geringen Luftfördermengen als Nebenwirkungen der Luftmengenförderleistung entstehen, durch die Strukturen mit den Kanälen mit Umlenkungen reduziert wird.

Die konstruktive Ausgestaltung der Labyrinthstruktur mit den Strukturen von Kanälen mit Umlenkungen in dem mindestens einen Labyrinthelement bewirkt, dass die Betriebsgeräusche derart geleitet, umgelenkt und reflektiert werden, dass am Gaseinlass des Verbindungselements diese Betriebsgeräusche im Schallpegel deutlich gegenüber dem Schallpegel am Gebläse reduziert sind. Die Dimensionierung des Verbindungselementes ist derart gewählt, dass ein Strömungsquerschnitt A_in am Gaseinlass des Verbindungselementes einem Strömungsquerschnitt A_out am mindestens einen Gasauslass des Verbindungselementes entspricht. Im Sinne der Erfindung sind dabei der Strömungsquerschnitt A_in am Gaseinlass und der Strömungsquerschnitt A_out am mindestens einen Gasauslass flächenmäßig in einer etwa gleichen Größenordnung. Ferner sind die Form des Strömungsquerschnittes A_in und die Form des Strömungsquerschnittes A_out unterschiedlich, im Wesentlichen gleich oder identisch. Die Form des jeweiligen Strömungsquerschnittes ist beispielsweise rund, rechteckig, quadratisch, oval oder elliptisch.

Mit der zuvor beschriebenen Dimensionierung des Verbindungselementes wird erreicht, dass am Gaseingang des Gebläsebeatmungsgerätes für die Einströmung von Luft aus der Umgebung durch das Verbindungselement und/oder das mindestens eine Labyrinthelement kein nennenswerter Druckabfall verursacht wird. Somit kann das Verbindungselement zusammen mit dem mindestens einen Labyrinthelement in Bezug auf einen Einfluss auf Steuerung und/oder Regelung des Gebläses im Gebläsebeatmungsgerät hinsichtlich Druckniveau und Fördermengen als weitgehend neutral angesehen werden.

Bevorzugte Ausführungsformen zeigen, wie die Dimensionierungen der Kanäle in dem Labyrinthelement bzw. Einlasskammer und/oder Auslasskammer des Labyrinthelements gestaltet werden können.

So kann eine Summe aller Strömungsquerschnitte der Kanäle in dem mindestens einen Labyrinthelement so gewählt werden, dass diese der Summe der Strömungsquerschnitte der mindestens einen Einlasskammer des mindestens einen Labyrinthelements entspricht.

So kann eine Summe aller Strömungsquerschnitte der Kanäle in dem mindestens einen Labyrinthelement so gewählt werden, dass diese der Summe der Strömungsquerschnitte der mindestens einen Auslasskammer des mindestens einen Labyrinthelements entspricht.

So kann die Summe der Strömungsquerschnitte der mindestens einen Einlasskammer des mindestens einen Labyrinthelements so gewählt werden, dass diese der Summe der Strömungsquerschnitte am mindestens einen Gaseinlass des Verbindungselementes entspricht.

Diese genannten Dimensionierungen der Strömungsquerschnitte haben sich in der praktischen Umsetzung als geeignete Orientierungshilfen erwiesen, um durch geeignete Ausgestaltungen von Einlasskammer und/oder Auslasskammer des Labyrinthelements die Reduzierung von Betriebsgeräuschen in Anpassung an die vom Gebläseantrieb erzeugten Frequenzbereiche der Geräusch- bzw. Schallemissionen bestmöglich zu erzielen

Ausführungsformen können verschiedene Konstellationen und Anzahlen von Labyrinthelementen oder Verschlusselementen in Anordnung an das Verbindungselement zeigen.

So kann in einer weiter bevorzugten Ausführungsform vorgesehen sein, dass mindestens zwei Labyrinthelemente oder mindestens ein Verschlusselement an dem Verbindungselement angeordnet sind. Ein Verschlusselement entspricht einem Labyrinthelement, in welchem keine Labyrinthstruktur enthalten ist, das Verschlusselement ist damit gleichsam ein leeres Raumvolumen, welches mittels der Reflexion und Umlenkung der Betriebsgeräusche im Zusammenspiel von Verbindungselement und dem Labyrinthelement bei der Reduzierung der Betriebsgeräusche mitwirkt.

Es lassen sich Ausführungsformen ausbilden, in denen zwei Labyrinthelemente einander gegenüberliegend am Verbindungselement angeordnet sein können. Weiterhin lassen sich Ausführungsformen ausbilden, in denen ein Verschlusselement und ein Labyrinthelement einander gegenüberliegend am Verbindungselement angeordnet sein können.

Es können Ausführungsformen mit unterschiedlichen zu Durchströmung verfügbaren Längen L des Verbindungselements und unterschiedlichen geometrischen Formen des Strömungsquerschnitts des Verbindungselementes ausgebildet werden.

Je nach Gestaltung und Platzsituation am Gaseingang des Gebläsebeatmungsgerätes können der Gaseinlass und/oder der Gasauslass im Verbindungselement relativ zu einander angeordnet sein.

In einer bevorzugten Ausführungsform ist die Anordnung von Verbindungselement und den beiden Labyrinthelementen, bzw. dem mindestens einen Lababyrinthelement und dem Verschlusselement derart ausgestaltet, dass die Strömungsmengen, die in den Gaseinlass des Verbindungselementes aus der Umgebung zuströmen und vom Verbindungselement an das Gebläsebeatmungsgerät ausströmen auf zwei parallelen Achsen strömen.

Der Gaseinlass und der Gasauslass des Verbindungselements können dabei auf der gleichen Raumachse angeordnet sein.

Der Gaseinlass und der Gasauslass des Verbindungselements können dabei zueinander mit einem horizontalen Versatz auf zwei zueinander parallelen Raumachsen angeordnet sein.

Der Gaseinlass und der Gasauslass des Verbindungselements können dabei zueinander mit einem vertikalen Versatz auf zwei zueinander parallelen Raumachsen angeordnet sein.

Der Gaseinlass und der Gasauslass des Verbindungselements können dabei zueinander mit einem horizontalen und vertikalen Versatz zueinander auf zwei zueinander parallelen Raumachsen angeordnet sein.

Je nach Gestaltung und Platzsituation am Gaseingang des Gebläsebeatmungsgerätes können der Gaseinlass und/oder der Gasauslass im Verbindungselement in Relation oder Winkelstellung zu dem mindestens einen Labyrinthelement oder den zwei gegenüberliegend angeordneten Labyrinthelementen angeordnet sein.

In einer bevorzugten Ausführungsform ist die Anordnung von Verbindungselement und den beiden Labyrinthelementen, bzw. dem mindestens einen Labyrinthelement und dem Verschlusselement derart ausgestaltet, dass die Strömungsmengen, die in den Gaseinlass des Verbindungselementes aus der Umgebung zuströmen und vom Verbindungselement an das Gebläsebeatmungsgerät ausströmen zu den Strömungsmengen, welche in die, bzw. das Labyrinthelement oder das Verschlusselement zu- und ausströmen, eine 90°- Konstellation zueinander ausbilden. Damit ergibt sich, dass die zuströmenden Strömungsmengen direkt nach der Einströmung durch den Gaseinlass um 90° seitlich umgelenkt werden und dann in das Labyrinthelement, bzw. das Verschlusselement einströmen und bei der Ausströmung aus dem Labyrinthelement, bzw. dem Verschlusselement um 90° seitlich umgelenkt werden und dann durch den Gasauslass ausströmen.

Je nach Gestaltung und Platzsituation am Gaseingang des Gebläsebeatmungsgerätes können dabei runde, rechteckige, quadratische, ovale, elliptische Querschnitte die Basis für die Ausgestaltung der Länge L des Verbindungselementes darbieten. In diesen Ausführungsformen ergeben sich dann für die Umsetzung in der Praxis gut anwendbare Dimensionierungen zwischen der Länge L des Verbindungselementes und dem Querschnitt des Verbindungselementes.

So kann in vorteilhafter Weise die zu einer Durchströmung verfügbare Länge L eines mit einem kreisrunden Querschnitt ausgebildeten Verbindungselements mindestens das Dreifache eines Innendurchmessers des mit einem kreisrunden Querschnitt ausgebildeten Verbindungselements betragen.

So kann die zu einer Durchströmung verfügbare Länge eines mit einem quadratischen Querschnitt ausgebildeten Verbindungselements mindestens das Dreifache einer Diagonale des Querschnitts des mit einem quadratischen Querschnitt ausgebildeten Verbindungselements betragen.

So kann die zu einer Durchströmung verfügbare Länge L eines mit einem rechteckigen Querschnitt ausgebildeten Verbindungselements mindestens das Dreifache einer Diagonale des Querschnitts des mit einem rechteckigen Querschnitt ausgebildeten Verbindungselements betragen.

So kann die zu einer Durchströmung verfügbare Länge L eines mit einem ovalen oder elliptischen Querschnitt ausgebildeten Verbindungselements mindestens das Dreifache der größeren Halbachse einer Ellipse oder mindestens das Dreifache eines Durchmessers einer im Wesentlichen runden Vergleichsgeometrie mit identischem Querschnitt des mit einem ovalen oder elliptischen Querschnitt ausgebildeten Verbindungselements betragen.

So kann das Verbindungselement derart ausgebildet sein, dass ein Quadrat der zu einer Durchströmung verfügbaren Länge L des Verbindungselementes mindestens das Neunfache des innerhalb des Verbindungselements gegebenen freien Strömungsquerschnittes beträgt.

So kann das Verbindungselement als ein Kanal mit einem weitestgehend symmetrischen Querschnitt mit einem Länge-zu-Breite-Verhältnis von ungefähr 1:1 ausgebildet sein.

Dabei können die Strömungsquerschnitte des mindestens einen Labyrinthelementes einen runden oder quadratischen Querschnitt und der Querschnitt des Gasauslasses einen runden oder quadratischen Querschnitt aufweisen.

Das Verbindungselement kann auch als ein Kanal mit einem weitestgehend unsymmetrischen Querschnitt mit einem Länge-zu-Breite-Verhältnis von ungefähr 2:1 ausgebildet sein.

Dabei können die Strömungsquerschnitte des mindestens einen Labyrinthelementes einen rechteckigen oder ovalen Querschnitt und der Querschnitt des Gasauslasses einen rechteckigen oder ovalen Querschnitt aufweisen.

In bevorzugten Ausführungsformen können die Labyrinthstrukturen den Rauminhalt der Labyrinthelemente zu mehr als 50%, vorzugsweise zu mehr als 60% ausfüllen.

Erfindungsgemäß weisen die Kanäle eine Mehrzahl von 90°-Umlenkungen und/oder eine Mehrzahl von 180°- Umlenkungen auf.

Diese genannten Dimensionierungen und Gestaltungen der Kanäle mit den Umlenkungen und der Füllung des Rauminhalts in den Labyrinthelementen haben sich in der praktischen Umsetzung als geeignet erwiesen, um die Reduzierung von Betriebsgeräuschen in Anpassung an die vom Gebläseantrieb erzeugten Frequenzbereiche der Geräusch- bzw. Schallemissionen bestmöglich zu erzielen.

In bevorzugten Ausführungsformen können die Labyrinthstrukturen derart in dem mindestens einen Labyrinthelement ausgebildet und angeordnet sein, dass im Übergang von einem Strömungsquerschnitt an der Einlasskammer auf Strömungsquerschnitte der Mehrzahl an parallelen Kanälen eine sprunghafte oder abrupte Reduzierung des Strömungsquerschnitts am jeweiligen Kanal der Mehrzahl an parallelen Kanälen um einen Unterschied von mindestens Faktor 2 gegeben ist. Sprunghafte oder abrupte Reduzierungen des Strömungsquerschnitts der Kanäle bewirken bei der Leitung von Geräuschen wirksame Reflexionen und Mehrfachreflexionen für die Geräusche und vermindern die damit verbliebenen Geräuschemissionen.

Zusammenfassend ergibt sich, dass die vorliegende Erfindung es ermöglicht, die vom Gebläsebeatmungsgerät an die Umgebung abgestrahlten Betriebsgeräusche wirksam zu reduzieren.

Im Folgenden werden anhand der Figuren beispielhafte Ausführungsbeispiele der Erfindung ohne eine Beschränkung auf die Allgemeinheit des Erfindungsgedankens näher erläutert.

Es zeigen:
die Figur 1 eine Anordnung zu einer Reduzierung von Geräuschen,
die Figur 2 ein Labyrinthelement nach der Figur 1 im Detail,
die Figur 3 eine Darstellung von Verbindungselementen mit zwei Labyrinthelementen.

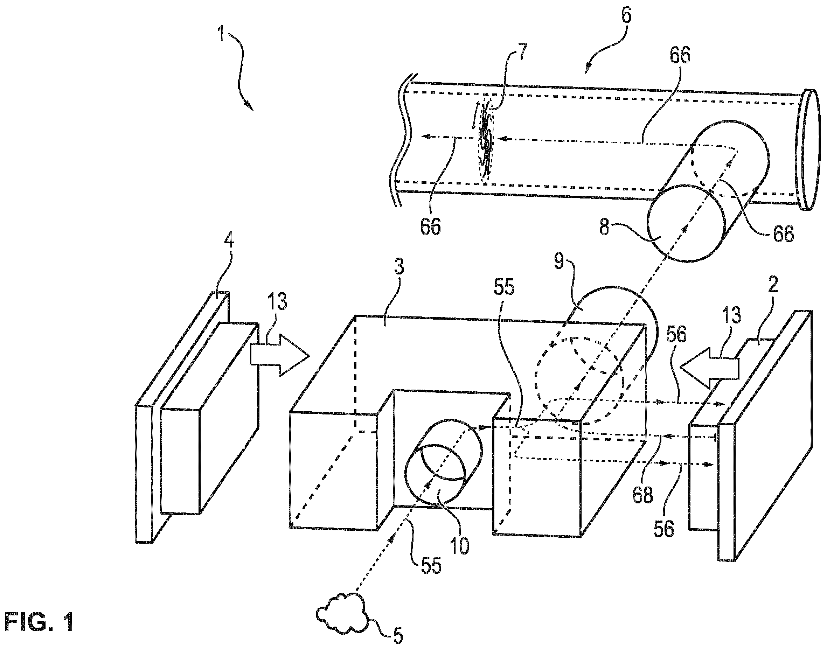

Die Figur 1 zeigt eine Anordnung 1 zu einer Reduzierung von Geräuschen für ein Medizingerät zu einer Beatmung eines Lebewesens mit einem schematisch in Form einer Gebläseantriebseinheit angedeuteten Gebläsebeatmungsgerät 6. Das Gebläsebeatmungsgerät 6 bzw. die Gebläseantriebseinheit 6 weist ein Radialgebläse 7 und einen Gaseingang 8 auf. Weitere Komponenten eines Gebläsebeatmungsgerätes 6 oder Beatmungsgerätes, wie pneumatische Verbindungen, Gehäuse, Ventile, Sensorik, Atemsystem, Schlauchleitungen, Beatmungsschläuche, Kontrolleinheit oder Anzeige- und Bedieneinheit sind in dieser Figur 1 nicht mit gezeigt, da sie für das Verständnis der Schallreduzierung nicht erläutert werden müssen, jedoch die zeichnerische Übersichtlichkeit beinträchtigen würden.

An den Gaseingang 8 des Gebläsebeatmungsgerätes 6 kann ein Verbindungselement 3 mittels eines Gasauslasses 9 pneumatisch angekoppelt werden. Elemente zur Abdichtung der Ankopplung zwischen Gaseingang 8 und Gasauslass 9 sind in üblicher Weise, z.B. in Form von O-Ringen vorhanden und dimensioniert, werden zur Wahrung der zeichnerischen Übersichtlichkeit jedoch nicht mit gezeigt. Das Radialgebläse 7 fördert Atemluftmengen 66 vom Verbindungselement 3 über den Gasauslass 9 und den Gaseingang 8 in ein Atemsystem des Gebläsebeatmungsgerätes 6. Das Gebläsebeatmungsgerät stellt diese Atemluftmengen 66 dann im Rahmen einer kontrollierten und/oder unterstützenden Beatmung einem Lebewesen zur Verfügung.

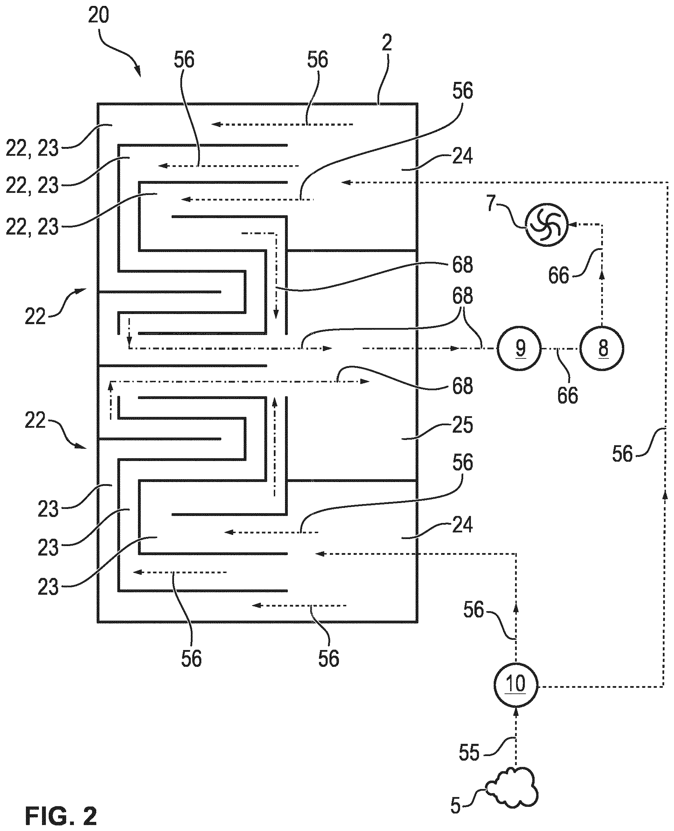

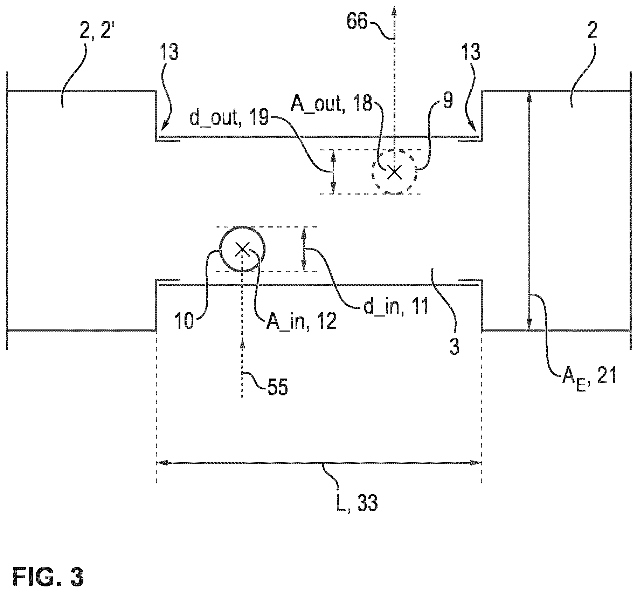

An das Verbindungselement 3 ist mindestens ein Labyrinthelement 2 bzw. 2' (Figur 3) mit einer Kanäle 23 (Figur 2) ausbildenden Labyrinthstruktur 22 (Figur 2), mit mindestens einer Einlasskammer (24) und mit mindestens einer Auslasskammer 25 (Figur 2) ankoppelbar.

Die Pfeildarstellungen 13 sollen die Ankopplung der Labyrinthelemente 2 bzw. 2' (Figur 3) an das Verbindungselement 3 verdeutlichen.

In dieser Figur 1 ist an dem dem Verbindungselement 3 gegenüberliegenden Labyrinthelement 2 ein Verschlusselement 4 gezeigt, welches keine Labyrinthstruktur aufweist und somit lediglich eine Funktion eines dichtenden seitlichen Verschlusses des Verbindungselementes 3 bereitstellt. Dichtelemente zwischen Verbindungselement 3, Labyrinthelement 2 und Verschlusselement 4 sind in üblicher Art und Weise vorgesehen, werden zur Wahrung der zeichnerischen Übersichtlichkeit jedoch in dieser Figur 1 nicht mit gezeigt.

Es sind allerdings auch verschiedenste weitere Varianten der Anordnung 1 zu einer Reduzierung von Geräuschen für ein Gebläsebeatmungsgerät 6 ausgestaltbar, welche mindestens zwei Labyrinthelemente 2, 2' (Figur 3), beispielsweise in einer gegenüberliegenden Anordnung mit mittig angeordnetem Verbindungselement 3 aufweisen.

Über einen Gaseinlass 10 gelangen aus einer Umgebung 5 Umgebungsluftmengen 55 in das Verbindungselement 3 hinein und werden als Einlassluftmengen 56 mindestens einer, in dem Labyrinthelement 2 angeordneten Einlasskammer 24 (Figur 2) bereitgestellt. Im Labyrinthelement 2 erfolgt die Führung der Umgebungsluftmengen 55 durch die Kanäle 23 (Figur 2) der Labyrinthstruktur 22 (Figur 2) und über mindestens eine Auslasskammer 25 (Figur 2) als Auslassluftmengen 68 wieder zurück in das Verbindungselement 3 und von dort über den Gasauslass 9 vom Verbindungselement 3 dann als Atemluftmengen 66 zum Gaseingang 8 des Gebläsebeatmungsgerätes 6. In der Figur 1 ist gezeigt, dass die Strömungsmengen 55 nach dem Gaseinlass 10 im Verbindungselement 3 aus der Umgebung 5 rechtwinklig als Strömungsmengen 56 in das Labyrinthelement 2 strömen. In der Figur 1 ist gezeigt, dass die Strömungsmengen 68 vom dem Labyrinthelement 2 im Verbindungselement 3 rechtwinklig als Strömungsmengen 68 in den Gasauslass 9 strömen.

In der Figur 1 sind der Gaseinlass 10 und Gasauslass 9 des Verbindungselements 3 in einer konstruktiven Situation beispielhaft mit einem horizontalen und vertikalen Versatz zueinander auf zwei zueinander parallelen Raumachsen angeordnet. Alternative konstruktive Situationen, in denen lediglich ein horizontaler Versatz oder nur ein vertikaler Versatz ausgestaltet sind, sind vom Kerngedanken der Ausgestaltung des Verbindungselementes 3 jedoch mit umfasst, jedoch zur Wahrung einer vereinfacht schematischen und übersichtlichen Darstellung nicht mit in der Figur 1 gezeigt. Geeignete Positionierungen des Verbindungselements 3 am Gaseingang 8 des Gebläsebeatmungsgerätes 6 ergeben sich aus Form und Dimensionierungen des Gebläsebeatmungsgerätes 6 mit dem Ziel einer möglichst raumsparenden und strömungsgünstigen Anordnung.

Details zu Komponenten und zur Funktionsweise des Labyrinthelementes 2 werden nachfolgend anhand der Figuren 2 und 3 näher erläutert. Gleiche Elemente in den Figuren 1, 2,3 sind in den Figuren 1, 2, 3 mit identischen Bezugsziffern bezeichnet.

Die Figur 2 zeigt in einer Darstellung 20 das Labyrinthelement 2 mit Labyrinthstruktur 22 und sich durch das Labyrinth ausbildende Kanäle 23, welche die Einströmung 56, Durchströmung und Ausströmung 68 von Luftmengen durch das Labyrinthelement 2 leiten. Die Einströmung 56 von Umgebungsluftmengen 55 aus der Umgebung 5 erfolgt in diesem Ausgestaltungsbeispiel nach der Figur 2 vom Verbindungselement 3 (Figur 1) her über den Gaseinlass durch zwei Einlasskammern 24. Die Ausströmung 68 von Auslassluftmengen zurück zu Verbindungselement 3 (Figur 1) und dann über den Gasauslass 9 des Verbindungselementes 3 (Figur 1) zum Gaseingang 8 eines Gebläsebeatmungsgerätes 6 hin bis zum Radialgebläse 7 erfolgt in diesem Ausgestaltungsbeispiel nach der Figur 2 mittels einer Auslasskammer 25.

Es sind allerdings auch Ausgestaltungen mit mehreren Einlasskammern und/ oder mehreren Auslasskammern möglich, die in dieser Figur 2 dargestellte Variante mit zwei Einlasskammern 24 und einer Auslasskammer 25 ist an die rechteckigen Außenkonturen des Labyrinthelementes 2 und die platzsparende Ausgestaltung der Zusammenfügung von Labyrinthelementen 2, 2' (Figur 1, Figur 3), des Verbindungselementes 3 (Figur 1) mit dem Gaseingang 8 des Gebläsebeatmungsgeräts 6 (Figur 1) zu einer Anordnung 1 (Figur 1) zu einer Reduzierung von Geräuschen am Gaseingang 8 eines Gebläsebeatmungsgeräts 6 (Figur 1) angepasst.

Die Figur 3 zeigt eine Darstellung 30 von Verbindungselement 3 und zwei Labyrinthelementen 2, 2'. Der Gaseinlass 10 ist mit einem Einlassdurchmesser d_in 11 und einem Einlassquerschnitt A_in 12 am Verbindungselement 3 schematisch gezeigt. Der Gasauslass 9 ist mit einem Auslassdurchmesser d_out 19 und einem Auslassquerschnitt A_out 18 am Verbindungselement 3 schematisch gezeigt. Der Abstand zwischen den zwei Labyrinthelementen 2, 2' ist als Länge L 33 schematisch gezeigt. Es ist weiterhin ein summarischer Querschnitt A_E 21 der Mehrzahl der Kanäle 23 (Figur 2) der Labyrinthelemente (2, 2') angedeutet. Zudem sind die Umgebungsluftmenge 55 am Gaseinlass 10 und die Atemluftmenge 66 am Gasauslass 9 des Verbindungselements 3 schematisch angedeutet.

Anhand dieser Figur 3 mit Referenzierungen zu den Figuren 1 und 2 sollen nun einige beispielhafte vorteilhafte Maßverhältnisse zwischen Strömungsquerschnitten A 12, 18, Durchmessern d 11, 19 und Längen L 33 etwas näher veranschaulicht werden.

Wesentlich ist beispielsweise, dass Einlassquerschnitt A_in 12, Auslassquerschnitt A_out 18 am Verbindungselement 3 und der summarische Querschnitt A_E 21 aller Kanäle 23 (Figur 2) der Labyrinthelemente (2, 2') in einer etwa gleichen Größenordnung sind.

Weiterhin ist eine Auslegung derart bevorzugt, dass ein Quadrat der zu einer Durchströmung verfügbaren Länge L 33 des Verbindungselementes 3 mindestens das Neunfache des innerhalb des Verbindungselements 3 gegebenen freien Strömungsquerschnittes aufweist.

Weiterhin ist es bei einem mit einem kreisrunden Durchmesser ausgebildeten Verbindungselement vorteilhaft, wenn das Verhältnis der Länge L 33 mindestens das Dreifache des zur Länge L 33 zugehörigen Innendurchmessers des Verbindungselementes 3 beträgt. Weiterhin ist vorteilhaft, wenn die Labyrinthstrukturen 22 (Figur 2) einen Rauminhalt der Labyrinthelemente 2, 2' zu mehr als 40%, vorzugsweise zu mehr als 50%, weiter bevorzugt zu mehr als 60% ausfüllen.

Diese Dimensionierungshilfen lassen sich mittels üblicher Flächenbe- und Umrechnungen auch auf andere Geometrien und Querschnittsformen des Verbindungselementes 3 übertragen, etwa auf ovale, elliptische, rechteckige oder quadratische Querschnittsformen des Verbindungselementes und von der runden Form abweichende Geometrien des Gaseinlasses 10 oder des Gasauslasses 9 des Verbindungselementes 3 wie auch auf Ausgestaltungen mit der quadratisch/-rechteckigen Formgebung der Labyrinthelemente 2, 2'.

### Bezugsziffernliste

- 1: Anordnung zu einer Reduzierung von Geräuschen
- 2, 2': Labyrinthelement mit Labyrinthstruktur
- 3: Verbindungselement
- 4: Verschlusselement, Deckel
- 5: Umgebung
- 6: Gebläsebeatmungsgerät, Gebläseantriebseinheit
- 7: Radialgebläse
- 8: Gaseingang eines Gebläsebeatmungsgerätes
- 9: Gasauslass des Verbindungselementes
- 10: Gaseinlass des Verbindungselementes
- 11: Durchmesser d_in des Gaseinlasses des Verbindungselementes
- 12: Querschnitt A_in des Gaseinlasses des Verbindungselementes
- 13: Zusammenfügung,
- 18: Querschnitt A_out des Gasauslasses des Verbindungselementes
- 19: Durchmesser d_out des Gasauslasses des Verbindungselementes
- 20: Darstellung der Labyrinthanordnung im Labyrinthelement
- 21: Summarischer Querschnitt A_E der Kanäle der Labyrinthelemente
- 22: Labyrinthstruktur
- 23: Kanäle
- 24: Einlasskammer am Labyrinthelement
- 25: Auslasskammer am Labyrinthelement
- 30: Darstellung von Verbindungselement und Labyrinthelement
- 33: Länge L des Verbindungselements
- 55: Umgebungsluftmengen aus der Umgebung
- 56: Einströmung in Einlasskammer am Labyrinthelement
- 66: Atemluftmengen aus Auslasskammer, Ausströmung
- 68: Auslassluftmenge aus Auslasskammer

## Patentansprüche

1. Anordnung (1) zu einer Reduzierung von Geräuschen an einem Gaseinlass eines Gebläsebeatmungsgerätes (6) mit einem Verbindungselement (3) und mit mindestens einem Labyrinthelement (2) mit einer Labyrinthstruktur (22),
- wobei die Labyrinthstruktur (22) einen Rauminhalt des mindestens einen Labyrinthelements (2) zu mehr als 40% ausfüllt,
- wobei die Labyrinthstruktur (22) eine Vielzahl von parallelen Kanälen (23) mit Umlenkungen in dem mindestens einen Labyrinthelement (2) ausbildet,
- wobei die parallelen Kanäle (23) zu einer Führung von Luftmengen (56, 68) und zu einer Beeinflussung einer Schallausbreitung ausgebildet sind,
- wobei die parallelen Kanäle (23) eine Mehrzahl von 90°- Umlenkungen und/oder eine Mehrzahl von 180°- Umlenkungen aufweisen,
- wobei das Verbindungselement (3) einen Gasauslass (9) aufweist, welcher mit einem Gaseingang (8) des Gebläsebeatmungsgerätes (6) verbindbar ist,
- wobei das Verbindungselement (3) einen Gaseinlass (10) zu einer Einströmung von Luftmengen (55) aus einer Umgebung (5) aufweist,
- wobei das mindestens eine Labyrinthelement (2) mindestens eine Einlasskammer (24) zu einer Aufnahme von Einlassluftmengen (56) vom Verbindungselement (3) aufweist,
- wobei das mindestens eine Labyrinthelement (2) mindestens eine Auslasskammer (25) zu einer Bereitstellung von Einlassluftmengen (68) an das Verbindungselement (3) aufweist,
- wobei das Verbindungselement (3) mit dem mindestens einen Labyrinthelement (2) derart verbunden ist, dass die Luftmengen (55) aus der Umgebung (5) in die Labyrinthstruktur (22) als Einlassluftmengen (56) einströmen, die Vielzahl an Kanälen (23) des mindestens einen Labyrinthelementes (2) durchströmen und als Auslassluftmengen (68) über das Verbindungselement (3) und den Gasauslass (9) des Verbindungselementes (3) als Atemluftmengen (66) in den Gaseingang (8) des Gebläsebeatmungsgerätes (6) einströmen,
- wobei ein Strömungsquerschnitt A_in (12) am Gaseinlass (10) des Verbindungselementes (3) einem Strömungsquerschnitt A_out (18) am mindestens einen Gasauslass (9) des Verbindungselementes (3) entspricht.

2. Anordnung nach Anspruch 1,
- wobei eine Summe aller Strömungsquerschnitte der Kanäle (23) in dem mindestens einen Labyrinthelement (2) der Summe der Strömungsquerschnitte der mindestens einen Einlasskammer (24) des mindestens einen Labyrinthelements (2) entspricht,
- wobei eine Summe aller Strömungsquerschnitte der Kanäle (23) in dem mindestens einen Labyrinthelement (2) der Summe der Strömungsquerschnitte der mindestens einen Auslasskammer (25) des mindestens einen Labyrinthelements (2) entspricht,
- wobei die Summe der Strömungsquerschnitte der mindestens einen Einlasskammer (24) des mindestens einen Labyrinthelements (2) der Summe der Strömungsquerschnitte am mindestens einen Gaseinlass (10) des Verbindungselementes (3) entspricht.

3. Anordnung nach Anspruch 1 oder Anspruch 2, wobei mindestens zwei Labyrinthelemente (2, 2') oder mindestens ein Verschlusselement (4) an dem Verbindungselement (3) angeordnet sind.

4. Anordnung nach Anspruch 1 oder Anspruch 2, wobei zwei Labyrinthelemente (2, 2') mit einer Labyrinthstruktur (22) einander gegenüberliegend an dem Verbindungselement (3) angeordnet sind.

5. Anordnung nach Anspruch 1 oder Anspruch 2, wobei mindestens ein Labyrinthelement (2) mit einer Labyrinthstruktur (22) und ein Verschlusselement (4) ohne eine Labyrinthstruktur einander gegenüberliegend an dem Verbindungselement (3) angeordnet sind.

6. Anordnung nach einem der vorhergehenden Ansprüche, wobei das Verbindungselement (3) derart ausgebildet ist, dass die zu einer Durchströmung verfügbare Länge L (33) eines mit einem kreisrunden Querschnitt ausgebildeten Verbindungselements (3) mindestens das Dreifache eines Innendurchmessers des mit einem kreisrunden Querschnitt ausgebildeten Verbindungselements (3) beträgt,
oder
die zu einer Durchströmung verfügbare Länge L (33) eines mit einem quadratischen Querschnitt ausgebildeten Verbindungselements (3) mindestens das Dreifache einer Diagonale des Querschnitts des mit einem quadratischen Querschnitt ausgebildeten Verbindungselements (3) beträgt,
oder
die zu einer Durchströmung verfügbare Länge L (33) eines mit einem rechteckigen Querschnitt ausgebildeten Verbindungselements (3) mindestens das Dreifache einer Diagonale des Querschnitts des mit einem rechteckigen Querschnitt ausgebildeten Verbindungselements (3) beträgt,
oder
die zu einer Durchströmung verfügbare Länge L (33) eines mit einem ovalen oder elliptischen Querschnitt ausgebildeten Verbindungselements (3) mindestens das Dreifache der größeren Halbachse einer Ellipse oder mindestens das Dreifache eines Durchmessers einer im Wesentlichen runden Vergleichsgeometrie mit identischem Querschnitt des mit einem ovalen oder elliptischen Querschnitt ausgebildeten Verbindungselements (3) beträgt.

7. Anordnung nach einem der vorhergehenden Ansprüche, wobei das Verbindungselement (3) derart ausgebildet ist, dass ein Quadrat der zu einer Durchströmung verfügbaren Länge des Verbindungselementes (3) mindestens das Neunfache des innerhalb des Verbindungselements (3) gegebenen freien Strömungsquerschnittes beträgt.

8. Anordnung nach einem der vorhergehenden Ansprüche,
wobei das Verbindungselement (3) als ein Kanal mit einem weitestgehend symmetrischen Querschnitt mit einem Länge-zu-Breite-Verhältnis von ungefähr 1:1 ausgebildet ist und wobei die Strömungsquerschnitte des mindestens einen Labyrinthelementes (2, 2')
einen runden oder quadratischen Querschnitt aufweisen und wobei der Querschnitt des Gasauslasses einen runden oder quadratischen Querschnitt aufweist
oder
wobei das Verbindungselement (3) als ein flach ausgebildeter Kanal mit einem unsymmetrischen Querschnitt mit einem Länge-zu-Breite-Verhältnis von mehr als 2:1 ausgebildet ist und wobei die Strömungsquerschnitte
des mindestens einen Labyrinthelementes (2, 2') einen rechteckigen oder ovalen Querschnitt aufweisen und wobei der Querschnitt des Gasauslasses einen rechteckigen oder ovalen Querschnitt aufweist.

9. Anordnung nach einem der vorhergehenden Ansprüche, wobei die Labyrinthstrukturen (22) den Rauminhalt der Labyrinthelemente (2, 2') zu mehr als 50%, vorzugsweise zu mehr als 60% ausfüllen.

10. Anordnung nach einem der vorangehenden Ansprüche, wobei die Labyrinthstrukturen derart in dem mindestens einen Labyrinthelement (2, 2') ausgebildet und angeordnet sind, dass im Übergang von einem Strömungsquerschnitt an der Einlasskammer (24) auf Strömungsquerschnitte der Mehrzahl an parallelen Kanälen (23) eine sprunghafte oder abrupte Reduzierung des Strömungsquerschnitts am jeweiligen Kanal der Mehrzahl an parallelen Kanälen (23) um einen Unterschied von mindestens Faktor 2 gegeben ist.

## Claims

1. Arrangement (1) for reducing noise at a gas inlet of a blower ventilator (6) having a connecting element (3) and having at least one labyrinth element (2) having a labyrinth structure (22),
- wherein the labyrinth structure (22) fills more than 40% of the volume of the at least one labyrinth element (2),
- wherein the labyrinth structure (22) forms a plurality of parallel channels (23) having deflections in the at least one labyrinth element (2),
- wherein the parallel channels (23) are designed to guide air quantities (56, 68) and to influence sound propagation,
- wherein the parallel channels (23) have a plurality of 90° deflections and/or a plurality of 180° deflections,
- wherein the connecting element (3) has a gas outlet (9) which can be connected to a gas input (8) of the blower ventilator (6),
- wherein the connecting element (3) has a gas inlet (10) for an inflow of air quantities (55) from an environment (5),
- wherein the at least one labyrinth element (2) has at least one inlet chamber (24) for receiving inlet air quantities (56) from the connecting element (3),
- wherein the at least one labyrinth element (2) has at least one outlet chamber (25) for providing inlet air quantities (68) to the connecting element (3),
- wherein the connecting element (3) is connected to the at least one labyrinth element (2) such that the air quantities (55) from the environment (5) flow into the labyrinth structure (22) as inlet air quantities (56), flow through the plurality of channels (23) of the at least one labyrinth element (2) and flow as outlet air quantities (68) via the connecting element (3) and the gas outlet (9) of the connecting element (3) into the gas input (8) of the blower ventilator (6) as breathing air quantities (66),
- wherein a flow cross section A_in (12) at the gas inlet (10) of the connecting element (3) corresponds to a flow cross section A_out (18) at the at least one gas outlet (9) of the connecting element (3).

2. Arrangement according to claim 1,
- wherein a sum of all flow cross sections of the channels (23) in the at least one labyrinth element (2) corresponds to the sum of the flow cross sections of the at least one inlet chamber (24) of the at least one labyrinth element (2),
- wherein a sum of all flow cross sections of the channels (23) in the at least one labyrinth element (2) corresponds to the sum of the flow cross sections of the at least one outlet chamber (25) of the at least one labyrinth element (2),
- wherein the sum of the flow cross sections of the at least one inlet chamber (24) of the at least one labyrinth element (2) corresponds to the sum of the flow cross sections at the at least one gas inlet (10) of the connecting element (3).

3. Arrangement according to claim 1 or claim 2, wherein at least two labyrinth elements (2, 2') or at least one closure element (4) are arranged on the connecting element (3).

4. Arrangement according to claim 1 or claim 2, wherein two labyrinth elements (2, 2') having a labyrinth structure (22) are arranged opposite one another on the connecting element (3).

5. Arrangement according to claim 1 or claim 2, wherein at least one labyrinth element (2) having a labyrinth structure (22) and a closure element (4) without a labyrinth structure are arranged opposite one another on the connecting element (3).

6. Arrangement according to any of the preceding claims, wherein the connecting element (3) is designed such that the length L (33), available for flow, of a connecting element (3) having a circular cross section is at least three times the inner diameter of the connecting element (3) having a circular cross section,
or
the length L (33), available for flow, of a connecting element (3) having a square cross section is at least three times the diagonal of the cross section of the connecting element (3) having a square cross section,
or
the length L (33), available for flow, of a connecting element (3) having a rectangular cross section is at least three times the diagonal of the cross section of the connecting element (3) having a rectangular cross section,
or
the length L (33), available for flow, of a connecting element (3) having an oval or elliptical cross section is at least three times the major semi-axis of an ellipse or at least three times the diameter of a substantially round comparable geometry having an identical cross section to the connecting element (3) having an oval or elliptical cross section.

7. Arrangement according to any of the preceding claims, wherein the connecting element (3) is designed such that a square of the length, available for flow, of the connecting element (3) is at least nine times the free flow cross section which is within the connecting element (3).

8. Arrangement according to any of the preceding claims,
wherein the connecting element (3) is designed as a channel having a largely symmetrical cross section having a length-to-width ratio of approximately 1:1 and wherein the flow cross sections of the at least one labyrinth element (2, 2')
have a round or square cross section and wherein the cross section of the gas outlet has a round or square cross section
or
wherein the connecting element (3) is designed as a flat channel having an asymmetrical cross section having a length-to-width ratio of more than 2:1 and wherein the flow cross sections of the at least one labyrinth element (2, 2') have a rectangular or oval cross section and wherein the cross section of the gas outlet has a rectangular or oval cross section.

9. Arrangement according to any of the preceding claims, wherein the labyrinth structures (22) fill the volume of the labyrinth elements (2, 2') more than 50%, preferably more than 60%.

10. Arrangement according to any of the preceding claims, wherein the labyrinth structures are formed and arranged in the at least one labyrinth element (2, 2') such that in the transition from a flow cross section at the inlet chamber (24) to flow cross sections of the plurality of parallel channels (23), there is a sudden or abrupt reduction of the flow cross section at the relevant channel of the plurality of parallel channels (23) by a difference of at least a factor of 2.

## Revendications

1. Agencement (1) destiné à réduire le bruit au niveau d'une admission de gaz d'un appareil respiratoire à soufflante (6), comportant un élément de raccordement (3) et au moins un élément à labyrinthe (2) comportant une structure en labyrinthe (22),
- dans lequel la structure en labyrinthe (22) remplit un volume de l'au moins un élément à labyrinthe (2) à plus de 40 %,
- dans lequel la structure en labyrinthe (22) forme une pluralité de canaux (23) parallèles comportant des déviations dans l'au moins un élément à labyrinthe (2),
- dans lequel les canaux (23) parallèles sont réalisés pour guider des quantités d'air (56, 68) et pour influencer une propagation du son,
- dans lequel les canaux (23) parallèles présentent une pluralité de déviations de 90° et/ou une pluralité de déviations de 180°,
- dans lequel l'élément de raccordement (3) présente une évacuation de gaz (9) qui peut être raccordée à une entrée de gaz (8) de l'appareil respiratoire à soufflante (6),
- dans lequel l'élément de raccordement (3) présente une admission de gaz (10) pour un écoulement d'entrée de quantités d'air (55) provenant d'un environnement (5),
- dans lequel l'au moins un élément à labyrinthe (2) présente au moins une chambre d'admission (24) pour recevoir des quantités d'air d'admission (56) provenant de l'élément de raccordement (3),
- dans lequel l'au moins un élément à labyrinthe (2) présente au moins une chambre d'évacuation (25) pour fournir des quantités d'air d'admission (68) à l'élément de raccordement (3),
- dans lequel l'élément de raccordement (3) est raccordé à l'au moins un élément à labyrinthe (2) de telle sorte que les quantités d'air (55) provenant de l'environnement (5) s'écoulent dans la structure en labyrinthe (22) en tant que quantités d'air d'admission (56), traversent la pluralité de canaux (23) de l'au moins un élément à labyrinthe (2) et s'écoulent en tant que quantités d'air d'évacuation (68) dans l'entrée de gaz (8) de l'appareil respiratoire à soufflante (6) par l'intermédiaire de l'élément de raccordement (3) et de l'évacuation de gaz (9) de l'élément de raccordement (3) en tant que quantités d'air de respiration (66),
- dans lequel une section transversale d'écoulement A_in (12) au niveau de l'admission de gaz (10) de l'élément de raccordement (3) correspond à une section transversale d'écoulement A_out (18) au niveau de l'au moins une évacuation de gaz (9) de l'élément de raccordement (3).

2. Agencement selon la revendication 1,
- dans lequel une somme de toutes les sections transversales d'écoulement des canaux (23) dans l'au moins un élément à labyrinthe (2) correspond à la somme des sections transversales d'écoulement de l'au moins une chambre d'admission (24) de l'au moins un élément à labyrinthe (2),
- dans lequel une somme de toutes les sections transversales d'écoulement des canaux (23) dans l'au moins un élément à labyrinthe (2) correspond à la somme des sections transversales d'écoulement de l'au moins une chambre d'évacuation (25) de l'au moins un élément à labyrinthe (2),
- dans lequel la somme des sections transversales d'écoulement de l'au moins une chambre d'admission (24) de l'au moins un élément à labyrinthe (2) correspond à la somme des sections transversales d'écoulement au niveau de l'au moins une admission de gaz (10) de l'élément de raccordement (3).

3. Agencement selon la revendication 1 ou la revendication 2, dans lequel au moins deux éléments à labyrinthe (2, 2') ou au moins un élément de fermeture (4) sont disposés sur l'élément de raccordement (3).

4. Agencement selon la revendication 1 ou la revendication 2, dans lequel deux éléments à labyrinthe (2, 2') comportant une structure en labyrinthe (22) sont disposés en vis-à-vis sur l'élément de raccordement (3).

5. Agencement selon la revendication 1 ou la revendication 2, dans lequel au moins un élément à labyrinthe (2) comportant une structure en labyrinthe (22) et un élément de fermeture (4) sans structure en labyrinthe sont disposés en vis-à-vis sur l'élément de raccordement (3).

6. Agencement selon l'une des revendications précédentes, dans lequel l'élément de raccordement (3) est réalisé de telle sorte que la longueur L (33) d'un élément de raccordement (3) réalisé avec une section transversale circulaire et disponible pour un écoulement traversant est égale à au moins trois fois un diamètre intérieur de l'élément de raccordement (3) réalisé avec une section transversale circulaire,
ou
la longueur L (33) d'un élément de raccordement (3) réalisé avec une section transversale carrée et disponible pour un écoulement traversant est égale à au moins trois fois une diagonale de la section transversale de l'élément de raccordement (3) réalisé avec une section transversale carrée,
ou
la longueur L (33) d'un élément de raccordement (3) réalisé avec une section transversale rectangulaire et disponible pour un écoulement traversant est égale à au moins trois fois une diagonale de la section transversale de l'élément de raccordement (3) réalisé avec une section transversale rectangulaire,
ou
la longueur L (33) d'un élément de raccordement (3) réalisé avec une section transversale ovale ou elliptique et disponible pour un écoulement traversant est égale à au moins trois fois le plus grand demi-axe d'une ellipse ou à au moins trois fois un diamètre d'une géométrie de comparaison sensiblement ronde comportant une section transversale identique de l'élément de raccordement (3) réalisé avec une section transversale ovale ou elliptique.

7. Agencement selon l'une des revendications précédentes, dans lequel l'élément de raccordement (3) est réalisé de telle sorte qu'un carré de la longueur de l'élément de raccordement (3) disponible pour un écoulement traversant est égal à au moins neuf fois la section transversale d'écoulement libre obtenue à l'intérieur de l'élément de raccordement (3).

8. Agencement selon l'une des revendications précédentes,
dans lequel l'élément de raccordement (3) est réalisé sous forme de canal comportant une section transversale largement symétrique comportant un rapport longueur/largeur d'environ 1:1, et dans lequel les sections transversales d'écoulement de l'au moins un élément à labyrinthe (2, 2')
présentent une section transversale ronde ou carrée et dans lequel la section transversale de l'évacuation de gaz présente une section transversale ronde ou carrée
ou
dans lequel l'élément de raccordement (3) est réalisé sous forme de canal plat comportant une section transversale asymétrique comportant un rapport longueur/largeur supérieur à 2:1 et dans lequel les sections transversales d'écoulement de l'au moins un élément à labyrinthe (2, 2') présentent une section transversale rectangulaire ou ovale et dans lequel la section transversale de l'évacuation de gaz présente une section transversale rectangulaire ou ovale.

9. Agencement selon l'une des revendications précédentes, dans lequel les structures en labyrinthe (22) remplissent le volume des éléments à labyrinthe (2, 2') à plus de 50 %, de préférence à plus de 60 %.

10. Agencement selon l'une des revendications précédentes, dans lequel les structures en labyrinthe sont réalisées et disposées dans l'au moins un élément à labyrinthe (2, 2') de telle sorte que, lors du passage d'une section transversale d'écoulement au niveau de la chambre d'admission (24) à des sections transversales d'écoulement de la pluralité de canaux (23) parallèles, une réduction brusque ou abrupte de la section transversale d'écoulement est obtenue d'une différence d'au moins un facteur 2 au niveau du canal respectif de la pluralité de canaux (23) parallèles.
